# EUROPÄISCHE PATENTANMELDUNG

(11) **EP 1 975 528 A2**
(43) Veröffentlichungstag der Anmeldung: **01.10.2008**
(21) Anmeldenummer: 08004576.8
(22) Anmeldetag: 12.03.2008
(51) Int. Cl.: F25C 1/00, F28G 9/00

(54) **Vorrichtung und Verfahren zum Desinfizieren von Eismaschinen, Eissilos und/oder Kanälen zum Transportieren von Eis**

(30) Priorität: 29.03.2007 DE 102007015584
(71) Anmelder: Maja-Maschinenfabrik Hermann Schill GmbH & Co. KG, 77694 Kehl-Goldscheuer (DE)
(72) Erfinder: Schill, Joachim, 77694 Kehl (DE); Roos, Joachim, 77694 Kehl (DE)
(74) Vertreter: Lucht, Silvia

(57) **Zusammenfassung**

Vorrichtung und Verfahren zum Desinfizieren von Eismaschinen, Eissitos und/oder Kanälen zum Transportieren von Eis mit einem Vorratsbehälter (8) zur Aufnahme eines Desinfektionsmittels, mit einer Druckleitung (7) zum Zuleiten eines Trägertluids unter Druck, mit mindestens einer Zerstäubungseinrichtung (6) zum Zerstäuben des Desinfektionsmittels und zur Erzeugung eines Gemischs aus Trägerfluid und Desinfektionsmittel und mit einem Einlass zum Einleiten des TrägerRuid-Desinfaktionsmfttel-Gemischs in die Eismaschine (1, 19, 23, 28, 32), das Eissilo (2, 20, 24, 33) oder die Eiskanäle (4. 21, 29, 34).

## Beschreibung

### Stand der Technik

Die Erfindung geht aus von einer Vorrichtung und einem Verfahren zum Desinfizieren von Eismaschinen, Eissilos und/ oder Eiskanälen zum Transportieren von Eis sowie von Eismaschinen und Eissilos, welche mit Vorrichtungen zum Desinfizieren ausgestattet sind.

Eismaschinen dienen dazu, aus beliebigen Flüssigkeiten Eis herzustellen. Das Eis kann dabei unterschiedliche Formen aufweisen wie beispielsweise dünne Blättchen, Würfel, Flocken, Flüssigeis oder ein Granulat aus Körnern. Als Flüssigkeit zur Herstellung des Eises wird häufig Wasser eingesetzt. Das aus Wasser produzierte Eis wird zur Herstellung von Nahrungsmitteln und zur Frischhaltung von Nahrungsmitteln beim Transport und bei der Lagerung eingesetzt. Auf diese Weise können beispielsweise Fleisch, Fisch oder Meerestiere gelagert und transportiert werden, ohne dass deren Qualität leidet. Eis in Form von dünnen Blättchen, welches auch als Scherbeneis bezeichnet wird, wird bei der Herstellung von Wurst eingesetzt. Neben Wasser können auch andere Flüssigkeiten wie beispielsweise Säfte, Soßen, Ei, Milch und Milchprodukte zu Eis verarbeitet werden. Das mittels einer Eismaschine hergestellte Eis wird bei größeren Anlagen in sogenannten Eissilos gesammelt, bevor es der weiteren Bearbeitung oder dem Einsatzort zugeführt wird. Die Eissilos sind meist mit großen Behältern ausgestattet, in welche das durch die Eismaschine produzierte Eis über Kanäle, welche auch als Eisschächte bezeichnet werden, geleitet wird. Die Eissilos sind häufig wärmegedämmt, um zu verhindern, dass das Eis während der Lagerung schmilzt.

Da das Eis für die Herstellung von Nahrungsmitteln eingesetzt wird, werden an die Eismaschine zur Herstellung des Eises und an das Eissilo zum Sammeln und Lagern des Eises besonders hohe Anforderungen hinsichtlich der Hygiene gestellt, Es besteht die Anforderung, sämtliche mit dem Eis in Berührung kommenden Oberflächen von Krankheitserregern, insbesondere Bakterien, Viren, Pilzen und Protozoen zu befreien. Hierzu ist eine Desinfektion der entsprechenden Oberflächen der Eismaschine, des Eissilos und der Kanäle, welche die Eismaschine mit dem Eissilo verbinden, in bestimmten Zeftabständen notwendig.

Aus der DE 4108911 A1 ist eine Eismaschine mit drehbarer Gefrierwalze und einer die Gefrierwalze umgebenden Wanne bekannt, weiche mit einer schaltbaren Reinigungsvorrichtung zum Spülen der Wanne und der Gefrierwalze ausgestattet ist. Die Reinigungsvorrichtung weist mehrere Sprühdüsen zum Besprühen der Gefrierwalze und der Wanne mit einem Reinigungsmittel auf. Als nachteilig erweist sich hierbei, dass lediglich diejenigen Teile der Eismaschine gereinigt werden, die sich innerhalb des Sprühkegels einer Sprühdüse befinden. Um die gesamte Gefrierwalze und die Wanne entsprechend zu reinigen, ist eine große Anzahl von Sprühdüsen notwendig. Ferner betrifft die Reinigung lediglich die der Herstellung des Scherbeneises dienenden Teile der Eismaschine. Die Vorrichtungen, welche dem Sammeln und Transportieren des Scherbeneises dienen, werden nicht gereinigt. Darüber hinaus wird zum Reinigen ein spezielle Reinigungsflüssigkeit verwendet, die über den Abfluss der Wanne aus der Eismaschine entfernt wird, und deren Reste durch anschließendes Spülen aus der Eismaschine beseitigt werden müssen. Die Reinigung kann daher nur stattfinden, solange kein Eis produziert wird und solange sich kein Eis und keine zu gefrierende Flüssigkeit in der Eismaschine befinden. Eine Reinigung während der Eisproduktion ist nicht möglich.

Aus der DE 19821284 A1 ist eine Scherbeneismaschine bekannt, die zur Reinigung, Desinfektion und Sterilisation des Scherbeneises mit mindestens einer UV-Lichtquelle ausgestattet ist. Diese kann sich an der Gefrierwalze, an der Einrichtung zum Trennen des Scherbeneises von der Mantelfläche der Gefrierwalze, an der Abtransporteinrichtung und an der Sammeleinrichtung zum Sammeln des hergestellten Eises befinden. Zwar ermöglicht die UV-Lichtquelle ein kontinuierliches Desinfizieren des Eises, jedoch ist die UV-Lichtquelle mit dem Nachteil von hohen Kosten verbunden. Die UV-Strahlen werden von der DNA der Bakterien und Pilze absorbiert. Dadurch wird die DNA-Struktur zerstört und die Mikroorganismen abgetötet. Dieser Vorgang hängt von der Intensität der UV-Strahlung ab. Zur Desinfektion sämtlicher mit dem Eis in Berührung tretender Oberflächen der Eismaschine, des Eissilos und der Kanäle ist daher eine direkte Beleuchtung dieser Oberflächen mit einer UV-Lichtquelle notwendig. Das Streulicht reicht für die Desinfektion aufgrund seiner geringen Intensität nicht aus. Daher wird eine große Anzahl von UV-Lichtquellen benötigt, weshalb das Verfahren und die Vorrichtung aufwendig und teuer sind.

Der Erfindung liegt daher die Aufgabe zugrunde, eine Vorrichtung zum Desinfizieren von Eismaschinen, Eissilos und Kanälen zum Transportieren von Eis zur Verfügung zu stellen, die kostengünstig hergestellt werden kann, eine zuverlässige Desinfizierung sämtlicher mit dem Eis in Berührung kommenden Oberflächen ermöglicht, und mit der eine Desinfizierung auch während der Eisproduktion durchgeführt werden kann, insbesondere solange sich Eis oder die, zu Eis zu gefrierende Flüssigkeit in der Eismaschine oder dem Eissilo befinden.

Die Erfindung und ihre Vorteile

Die erfindungsgemäße Vorrichtung und das erfindungsgemäße Verfahren zeichnen sich dadurch aus, dass ein Desinfektionsmittel mit einer Zerstäubungseinrichtung in kleine Partikel zerstäubt und mit einem Trägerfluid gemischt wird um ein Trägerfluid-Desinfektionsmittel-Gemisch zu erhalten. Anschließend wird das Trägerftuid-Desinfektionsmittel-Gemisch in die Eismaschine, das Eissilo und/ oder die Eiskanäle zum Transportieren von Eis eingeleitet. Hierzu ist die Eismaschine, das Eissilo oder der Eiskanal mit einem Einlass für das Trägerfluid-Desinfektionsmittel-Gemisch ausgestattet. Handelt es sich bei der Zerstäubungseinrichtung um eine Zerstäubungsdüse, so dient diese nicht nur dem Zerstäuben des Desinfektionsmittels sondern auch dem Mischen von Trägerfluid und zerstäubten Desinfektionsmittel und dem Einleiten des Trägerfluid-Desinfektionsmittel-Gemischs in die Eismaschine, das Eissilo oder den Eiskanal.

Wird als Trägerfluid Luft verwendet, so bildet sich bei diesem Vorgang ein Aerosol aus Luft und Partikeln des Desinfektionsmittels. Ein Aerosol ist ein dynamisches System und unterliegt ständigen Änderungen durch Kondensation von Dämpfen an bereits vorhandenen Partikeln, Verdampfen flüssiger Bestandteile der Partikel, Koagulation kleiner Teilchen zu großen und Abscheidung von Teilchen an umgebenden Gegenständen. Das Trägerfluid-Desinfektionsmittef-Gerrüsch verhält sich wie ein Gas und breitet sich in der gesamten Eismaschine, dem gesamten Eissilo oder dem gesamten Kanal zum Transport des Eises aus. Hierzu genügen eine oder einige wenige Zerstäubungsdüsen. Ferner lagern sich die Partikel des Gemisches an den Krankheitserregern an. Dabei werden die Krankheitserreger aufgrund der Wechselwirkung mit dem Desinfektionsmittel unschädlich gemacht.

Die mit Gasen vergleichbare Eigenschaft von Aerosolen wird durch den kleinen Durchmesser bedingt. Reduziert sich der Durchmesser, so verringern sich das Volumen und die Masse in der dritten Potenz. Die Querschnittsfläche verringert sich dagegen nur um die zweite Potenz. Die SinKgeschwindigkeit der Partikel hängt von ihrer Gravitationskraft und dem Luftwiderstand ab- Während die Gravitationskraft durch die Masse der Partikel bestimmt wird, hängt die Luftreibung von der Querschnittsfläche und der Geschwindigkeit ab. Bei einer Halbierung des Partikeldurchmessers nimmt die Sinkgeschwindigkeit um den Faktor 0,7 ab. Daraus resultiert, dass sich das Trägerfluid-Desinfektionsmittel-Gemisch umso besser in dem zu desinfizierenden Raum verteilt, je kleiner der Durchmesser der Partikel ist. Eine kritische Grenze bildet dabei der Durchmesser von 200µm. Unterhalb dieses Durchmessers verteilen sich die Partikel ähnlich wie ein Gas in dem zu desinfizierenden Volumen. Im Unterschied zu den bekannten Reinigungsvorrichtungen müssen die zu desinfizierenden Teile der Eismaschine oder des Eissilos nicht mehr in den Sprühkegel der Zerstäubungsdüse eingebracht werden. Da sich das Trägerfluid-Desinfektionsmittel-Gemisch vergleichbar mit einem Gas in dem betreffenden Volumen verteilt, genügt ein Einlass oder genügen einige wenige Einlässe um das gesamte Volumen mit dem Gemisch auszufüllen und die in dem Volumen angeordneten Teile zu desinfizieren.

Das Desinfektionsmittel muss der Anforderung genügen, dass es Krankheitserreger abtötet. Werden zusätzlich dazu auch die Dauerformen der Krankheitserreger, wie beispielsweise Sporen, beseitigt, so führt dies zu einer Sterilisation. Bei der Wahl des Desinfektionsmittels muss außerdem das Einsatzgebiet des mit der Eismaschine hergestellten Eises berücksichtigt werden. Da eine Desinfektion auch während des Betriebs des Eismaschine und des Sammelns von Eis in dem Eissilo stattfindet, darf das Desinfektionsmittel die Qualität des Eises nicht nachhaltig beeinflussen.

Um das Einbringen möglichst kleiner Partikeln des Desinfektionsmittels in das Trägerfluid zu begünstigen, wird das Trägerfluid unter Druck an einer Zuführung des Desinfektionsmittels vorbeigeführt. Dabei werden kleine Partikel des Desinfektionsmittels mitgenommen und in dem Trägerfluid in Strömungsrichtung transportiert. Der Druck liegt oberhalb des Atmosphärendrucks und beträgt typischerweise 0,5 bis 5,0 bar. Zum Druckaufbau wird beispielsweise ein Kompressor, eine Gasflasche, eine Pumpe oder eine bauseitige Druckluftanlage verwendet.

Als Trägerfluid eignen sich grundsätzlich Gase und Flüssigkeiten. Besonders kostengünstig ist der Einsatz von Luft als Trägerfluid. Darüber hinaus können andere Gase oder Wasserdampf als Trägerfluid eingesetzt werden. Wasserdampf hat den Vorteil, dass er aufgrund seiner höheren Temperatur das Desinfektionsmittel entsprechend erwärmt, was zu einer höheren Effizienz des Desinfektionsmittels beim Abtöten von Krankheitserregern führt. Im Vergleich zu Luft ist jedoch das Zurverfügungstellen von Wasserdampf unter Druck mit etwas größerem Aufwand verbunden.

Nach einer vorteilhaften Ausgestaltung der Erfindung ist die Zerstäubungseinrichtung eine Zerstäubungsdüse. Diese weist einen Anschluss für die Druckleitung, einen Einlass für das Desinfektionsmittel und eine Düsenöffnung zum Expandieren des Trägerfluid-Desinfektionsmittel-Gemischs in die Eismaschine, das Eissilo oder den Eiskanal auf. Vorteilhafterweise mündet die Druckleitung in einen Strömungskanal. Die Düsenöffnung ist an dem der Druckleitung abgewandten Ende des Strömungskanals angeordnet. Über eine Zuleitung oder eine Dosiereinrichtung ist der Vorratsbehälter des Desinfektionsmittels mit dem Strömungskanal verbunden. Bei Strömen des Trägerfluids in dem Strömungskanal reißt das Trägerfluid Partikel des Desinfektionsmittels mit. In Abhängigkeit von dem Druck des Trägerfluids und der Menge an zur Verfügung gestelltem Desinfektionsmittel stellt sich ein Mischungsverhältnis von Trägerfluid und Desinfektionsmittel ein. Ein zusätzliches Mischen oder Dosieren ist in diesem Fall nicht notwendig.

Nach einer weiteren vorteilhaften Ausgestaltung der Erfindung ist der Strömungskanal mit einem Hindernis zur Erzeugung von Verwirbelungen ausgestattet. Dabei kann es sich beispielsweise um ein Hindernis handeln, welches in den Strömungskanal quer zur Strömungsrichtung hineinragt. Die laminare Strömung des Trägerfluids wird dadurch in eine turbulente Strömung umgewandelt. Diese begünstigt den Vorgang des Mischens und Zerstäubens von Desinfektionsmittel in dem Trägerfluid.

Nach einer weiteren vorteilhaften Ausgestaltung der Erfindung ist die Zerstäubungseinrichtung ein Kontaktzerstäuber, ein Rieselzerstäuber, ein Scheibenzerstäuber, ein Dampfzerstäuber, ein Ultraschallzerstäuber oder ein Klingenburg-Zerstäuber. Bei einem Kontakt- oder Rieselzerstäuber wird eine poröse Oberfläche mit dem Desinfektionsmittel berieselt. Das Trägerfluid strömt an der porösen Oberfläche vorbei und nimmt kleine Partikel des Desinfektionsmittels mit. Der Vorgang wird durch die Verdunstung des Desinfektionsmittels an der porösen Oberfläche begünstigt. Bei einem Scheibenzerstäuber wird das Desinfektionsmittel auf eine rotierende Scheibe aufgetragen. Aufgrund der Zentrifugalkraft werden kleine Partikel des Desinfektionsmittels als feiner Nebel von der Scheibe nach außen geschleudert und von dem strömenden Trägerfluid mitgenommen. Bei einem Dampfzerstäuber wird flüssiges Desinfektionsmittel so stark erwärmt, dass es in den gasförmigen Zustand übergeht. Hierzu werden auch Elektrodensysteme eingesetzt, die zur Erwärmung des Desinfektionsmittels dessen Leitfähigkeit ausnutzen. Das gasförmige Desinfektionsmittel wird in das strömende Trägerfluid eingeleitet. Bei einem Ultraschallzerstäuber wird eine Membran oder Platte in hochfrequente Schwingungen versetzt. Diese Schwingungen werden auf das flüssige Desinfektionsmittel übertragen. Dadurch werden kleine Partikel aus dem flüssigen Desinfektionsmittel herausgeschlagen, welche durch das strömende Trägerfluid mitgenommen werden. Bei einem Klingenberg-Zerstäuber werden in einem Wirbelgitter stabile Längswirbel erzeugt. In die Zentren der Wirbel wird flüssiges Desinfektionsmittel mit hohem Druck eingedüst, Durch die Wirbel wird das Desinfektionsmittel in kleinste Partikel zerstäubt. Das strömende Trägerfluid nimmt die kleinen Partikel mit.

Nach einer weiteren vorteilhaften Ausgestaltung der Erfindung ist die Zerstäubungseinrichtung mit einer Einrichtung zum elektrostatischen Aufladen des zerstäubten Desinfektionsmittels ausgestattet. Ferner werden die desinfizierenden Oberflächen der Eismaschine, des Eissilos oder der Eiskanäle elektrostatisch aufgeladen, so dass die Oberflächen die Partikel des Desinfektionsmittels elektrostatisch anziehen. Dabei ist zu beachten, dass die Partikel positiv und die Oberflächen negativ geladen sind oder umgekehrt.

Nach einer weiteren vorteilhaften Ausgestaltung der Erfindung ist der Vorratsbehälter mit Wasserstoffperoxid als Desinfektionsmittel ausgestattet. Wasserstoffperoxid hat den Vorteil, dass es in jedem Verhältnis mit Wasser mischbar ist und bei Raumtemperatur in Wasser und Sauerstoff zerfällt. Es ist ein starkes Oxidationsmittel, eine schwache Säure und wirkt bei vielen Mikroorganismen stark toxisch. Bei einer Konzentration von weniger als 8% stellt das Wasserstoffperoxid keine Gefährdung für die mit dem Desinfektionsmittel in Berührung tretenden Personen dar. Beim Zerfall von Wasserstoffperoxid in Wasser und Sauerstoff tritt die desinfizierende Wirkung ein. Diese Wirkung beruht auf dem beim Zerfall entstehenden reaktiven atomaren Sauerstoff. Dieser führt zu einer oxidativen Beschädigung von Zellbestandteilen der Mikroorganismen, insbesondere durch oxidative Quervemetzung prolinreicher Zellwandproteine und durch Inaktivierung katalytischer Cysteinreste in aktiven Zentren von Enzymen. Dies führt zur Abtötung der Zellen der Mikroorganismen. Krankheitserreger werden daher in zuverlässiger Weise unschädlich gemacht. Dabei wird ausgenutzt, dass sich die Partikel des fein zerstäubten Wasserstoffperoxids an den Krankheitserregern anlagern, wodurch eine Wechselwirkung zwischen den Krankheitserregern und dem Wasserstoffperoxid mit der Folge der Abtötung der Krankheitserreger garantiert ist.

Wasserstoffperoxid zeichnet sich gegenüber anderen Desinfektionsmitteln dadurch aus, dass es in Wasser und Sauerstoff zerfällt und daher bedenkenlos und ohne Einschränkung bei Eismaschinen und Eissilos eingesetzt werden kann, die der Lebensmittelherstellung und der Lebensmittelbearbeitung dienen, Der Desinfektionsvorgang kann daher auch während der Eisproduktion und zu Zeitpunkten stattfinden, zu denen sich Eis und die gefrierende Flüssigkeit in der Eismaschine oder dem Eissilo befinden. Ein zusätzlicher Spülvorgang nach der Desinfektion zum Beseitigen des Desinfektionsmittels aus der Eismaschine und dem Eissilo ist nicht notwendig. Die nach dem Desinfektionsvorgang in der Eismaschine oder dem Eissilo verbleibenden Zerfallsprodukte Sauerstoff und Wasser beeinträchtigen die Qualität des Eises nicht. Das Eis genügt den erhöhten Anforderungen, weiche in der Lebensmittelindustrie gelten.

Neben Wasserstoffperoxid können alternativ oder kumulativ weitere Desinfektionsmittel verwendet werden. Eine Lösung mit einer geringen Konzentration an Silberpartikeln eignet sich ebenfalls. Zur Anwendung im Bereich der Lebensmittelindustrie ist die Konzentration so gering zu wählen, dass sie sich nicht nachteilig auf die Qualität des Eises auswirkt. Ferner können Alkohole als Desinfektionsmittel eingesetzt werden.

Nach einer weiteren vorteilhaften Ausgestaltung der Erfindung ist der Vorratsbehälter für das Desinfektionsmittel mit der Zerstäubungsdüse über eine Desinfektionsmittel-Zuleitung verbunden. Bei dem Vorratsbehälter kann es sich beispielsweise um einen Kanister handeln, aus dem das Desinfektionsmittel über eine Sauglanze entnommen wird. Bei der Desinfektionsmittel-Zuleitung handelt es sich um eine übliche Flüssigkeitsleitung, beispielsweise einen Schlauch oder ein Rohr, die die Sauglanze mit der Zerstäubungsdüse verbindet. Befindet sich dabei der Vorratsbehälter auf demselben Niveau wie die Zerstäubungsdüse, so gelangt das Desinfektionsmittel allein aufgrund seiner Gewichtskraft ohne äußere Hilfsmittel von dem Vorratsbehälter zu der Düse. Durch Veränderung des Niveaus des Vorratsbehälters relativ zur Zerstäubungsdüse, kann die Geschwindigkeit, mit der das Desinfektionsmittel vom Vorratsbehälter zur Zerstäubungsdüse transportiert wird, beeinflusst werden. Dies wiederum hat einen Einfluss auf die Menge an Desinfektionsmittel in dem Trägerfluid-Desinfektionsmittel-Gemisch, das in der Zerstäubungsdüse hergestellt wird.

Nach einer weiteren vorteilhaften Ausgestaltung der Erfindung ist an der Zerstäubungsdüse oder an der Desinfektionsmittel-Zuleitung eine Heizeinrichtung angeordnet. Dabei kann es sich beispielsweise um einen Durchlauferhitzer handeln. Ferner können die Düse oder die Desinfektionsmittelzuleitung mit einem Heizdraht oder einer Heizmanschette ausgestattet sein. Um zu verhindern, dass das Desinfektionsmittel vor der Zerstäubung erneut abkühlt, ist dafür Sorge zu tragen, dass die Heizeinrichtung möglichst nahe an der Zerstäubungsdüse angeordnet ist. Bei einer Erwärmung des Desinfektionsmittels auf eine Temperatur zwischen 40 und 55 Grad Celcius wird eine deutlich höhere Effizienz des Desinfektionsmittels beim Abtöten von Krankheitserregern erreicht.

Nach einer weiteren vorteilhaften Ausgestaltung der Erfindung ist an die Druckleitung zum Zuführen des Trägerfluids eine Heizeinrichtung angeordnet. Diese kann alternativ oder kumulativ zu der Heizeinrichtung an der Desinfektionsmittel-Zuleitung oder der Zerstäubungsdüse vorgesehen sein. Sie führt ebenfalls zu einer Erwärmung des Desinfektionsmittels und damit zu einer Steigerung der Effizienz des Desinfektionsvorgangs.

Nach einer weiteren vorteilhaften Ausgestaltung der Erfindung ist die Vorrichtung mit einer Dosiereinrichtung zum Dosieren des Desinfektionsmittels in das Trägerfluid ausgestattet. Eine derartige Dosiereinrichtung kann beispielsweise mit einem Dosierelement ausgestattet sein, das eine vorgegebene Menge an Desinfektionsmittel aus dem Vorratsbehälter entnimmt und in das unter Druck strömende Trägerfluid einführt. Zu diesem Zweck kann das Dosierelement beispielsweise einen Hohlraum aufweisen, weicher in dem Vorratsbehälter mit flüssigem Desinfektionsmittel gefüllt wird. In bevorzugter Weise weist der Hohlraum einen Einlass und einen Auslass auf. Das Dosierelement wird in dem unter Druck strömenden Trägerfluid derart ausgerichtet, dass der Einlass und der Auslass in Strömungsrichtung ausgerichtet sind. Das strömende Trägerfluid reißt das in dem Hohlraum befindliche Desinfektionsmittel mit.

Nach einer weiteren vorteilhaften Ausgestaltung der Erfindung ist die Vorrichtung mit einer Mischeinrichtung zum Mischen des Desinfektionsmittels in dem Trägerfluid ausgestattet. Dabei kann es sich beispielsweise um eine spezielle Mischkammer handeln, die zusätzlich mit Vorrichtungen ausgestattet ist, die eine Verwirbelung des Trägerfluids und damit eine Durchmischung von Trägerfluid und Desinfektionsmittel bewirken.

Eine erfindungsgemäße Eismaschine oder ein erfindungsgemäßes Eissilo ist derart mit der erfindungsgemäßen Vorrichtung zum Desinfizieren ausgestattet, dass die mindestens eine Zerstäubungseinrichtung in das Gehäuse integriert ist. Der Einlass für das Trägerfluid-Desinfektionsmittel-Gemisch befindet sich vorteilhafter Weise an einem Gehäuseelement, das den Innenraum der Eismaschine, des Eissilos und/ oder der Eiskanäle begrenzt. Damit kann das feinzerstäubte Trägerfluid-Desinfektionsmittel-Gemisch in das Innere der Eismaschine, des Eissilos oder des Eiskanals eingeleitet werden. Die übrigen Teile der Vorrichtung zum Desinfizieren befinden sich außerhalb des zu reinigenden Volumens. Sie sind jedoch vorteilhafterweise in das die Eismaschine, das Eissilo oder den Eiskanal nach außen abgrenzende Gehäuse integriert.

Weitere Vorteile und vorteilhafte Ausgestaltungen der Erfindung sind der nachfolgenden Beschreibung, der Zeichnung und den Ansprüchen zu entnehmen.

### Zeichnung

in der Zeichnung ist ein Ausführungsbeispiel einer Vorrichtung zum Desinfizieren eines Eisschachtes und eines Eissilos dargestellt. Ferner zeigt die Zeichnung verschiedene Ausführungs beispiele einer Eismaschine mit Eisschacht und Eissilo, welche mit einer erfindungsgemäßen Vorrichtung zum Desinfizieren ausgestattet sind. Es zeigen:
- Figur 1: Aufbau einer Vorrichtung zum Desinfizieren eines Eisschachtes und eines Eissilos,
- Figur 2: erstes Ausführungsbeispiel einer Eismaschine mit Eisschacht und Eissilo in einer perspektivischen Darstellung,
- Figur 3: Eismaschine mit Eisschacht und Eissilo gemäß Figur 2 in einer Seitenansicht,
- Figur 4: Eismaschine mit Eisschacht und Eissilo gemäß Figur 2 in einer Ansicht von vorne,
- Figur 5: Eismaschine mit Eisschacht und Eissilo gemäß Figur 2 in einer Ansicht von oben,
- Figur 6: zweites Ausführungsbeispiel einer Eismaschine mit Eisschacht und Eissilo in einer Seitenansicht,
- Figur 7: drittes Ausführungsbeispiel einer Eismaschine mit Eisschacht in einer Ansicht von vorne
- Figur 8: viertes Ausführungsbeispiel einer Eismaschine mit Eisschacht und Eissilo in einer Ansicht von vorne,
- Figur 9: Eismaschine mit Eisschacht und Eissilo gemäß Figur 8 in einer Seitenansicht.

### Beschreibung der Ausführungsbeispiele

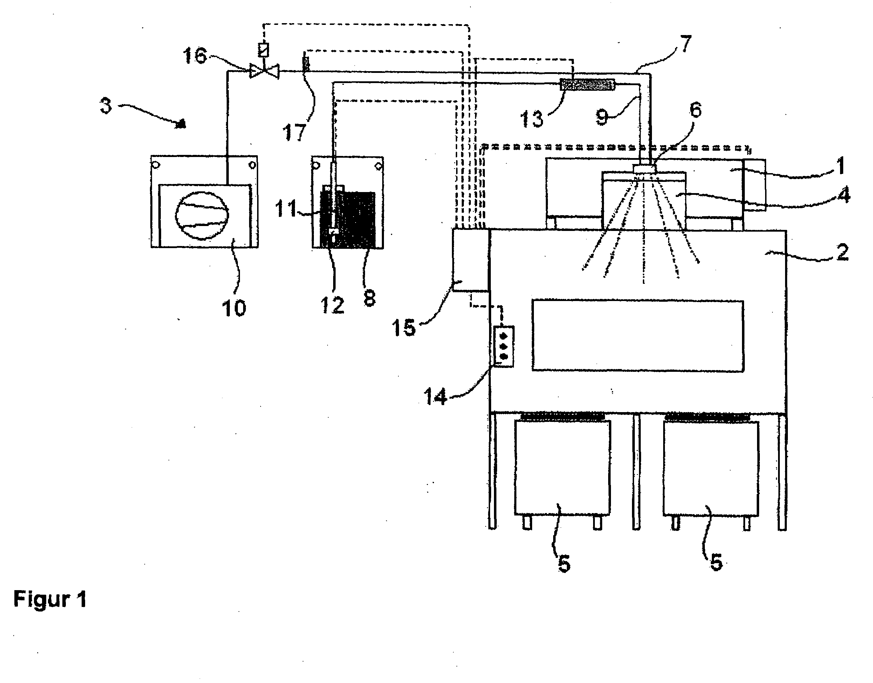

In Figur 1 ist der prinzipielle Aufbau einer Eismaschine 1, einem Eissilo 2 und einer Vorrichtung 3 zum Desinfizieren der Eismaschine 1 und des Eissilos 2 dargestellt. Bei der Eismaschine 1 handelt es sich um eine Scherbeneismaschine, bei der eine rotierende Gefrierwalze in eine teilweise mit Wasser gefüllte Wanne eintaucht. Die sich auf der Oberfläche der Gefrierwalze bildende Eisschicht wird mit Hilfe eines Schabers kontinuierlich abgelöst. Die Gefrierwalze, die Wanne und der Schaber befinden sich in dem Gehäuse der Eismaschine 1 und sind in der Zeichnung nicht erkennbar. Das von der Oberfläche der Gefrierwalze abgelöste Scherbeneis fällt durch einen als Eiskanal dienenden Eisschacht 4 in das Eissilo 2 und wird dort den beiden Eistransportwagen 5 zugeführt. Mittels dieser Eissilos wird das durch die Eismaschine 1 hergestellte Eis gesammelt und mit den Eistransportwagen der weiteren Verarbeitung oder dem Einsatzort zugeführt. Die Vorrichtung 3 zum Desinfizieren besteht im Wesentlichen aus einer Zerstäubungsdüse 6, einer Druckleitung 7 zum Zuleiten von Druckluft, einem Vorratsbehälter 8 mit Wasserstoffperoxid und einer Zuleitung 9, weiche den Vorratsbehälter 8 mit der Zerstäubungsdüse 6 verbindet. Die Druckleitung 7 mündet ebenfalls in die Zerstäubungsdüse 6. Die Druckluft wird mittels eines Druckluftkompressors 10 erzeugt. Das als Desinfektionsmittel dienende Wasserstoffperoxid wird mittels einer Sauglanze 11 aus dem Vorratsbehälter 8 entnommen und der Zuleitung 9 zugeführt. Da sich die Zerstäubungsdüse 6 und der Vorratsbehälter 8 zumindest näherungsweise auf demselben Niveau befinden, wird das Wasserstoffperoxid ohne weitere Hilfsmittel zu der Zerstäubungsdüse transportiert. Ein Schwimmerschalter 12 stellt fest, ob genügend Wasserstoffperoxid in dem Vorratsbehälter vorhanden ist. Fällt das Niveau unter einen vorgegebenen Grenzwert, so wird dem Benutzer der Mangel angezeigt. An der Zuleitung 9, welche den Vorratsbehälter 8 mit der Zerstäubungsdüse 6 verbindet, ist als Heizeinrichtung ein Durchlauferhitzer 13 angeordnet. Er erwärmt das der Zerstäubungsdüse zugeführte Wasserstoffperoxid, so dass dieses mit einer Temperatur von circa 40 bis 50 grad Celsius in den Eisschacht 4 eingeleitet wird.

Die Zerstäubungsdüse ist im oberen Bereich des Eisschachtes 4 angeordnet und nach unten ausgerichtet. Das sich in der Zerstäubungsdüse 6 mit der Druckluft vermischende und fein zerstäubte Wasserstoffperoxid wird in den Eisschacht 4 eingeleitet und breitet sich aufgrund seiner mit einem Gas vergleichbaren pysikalischen Eigenschaften im gesamten Volumen des Eisschachtes 4 und des Eissilos 2 aus.

An dem Gehäuse des Eissilos 2 ist eine Bedieneinrichtung 14 für die Vorrichtung zum Desinfizieren angeordnet. An diesem Bedienteil kann der Benutzer entweder den Beginn und das Ende eines Desinfektionsvorgangs manuell vorgeben oder eine automatische Desinfizierung in von ihm gewählten zeitlichen Abständen veranlassen. Die Bedieneinrichtung 14 ist mit einer Steuerungseinrichtung 15 verbunden. Diese betätigt ein Magnetventil 16 und einen Druckschalter 17 an der Druckleitung 7, wenn der Vorgang des Desinfizierens gestartet wird. Ferner wird die Sauglanze 11 aktiviert um Wasserstoffperoxid aus dem Vorratsbehälter 8 anzusaugen. Auf Wunsch des Benutzers wird der Durchlauferhitzer 13 zum Erwärmen des Wasserstoffperoxids eingeschaltet. Die Steuerungseinrichtung 15 ist außerdem mit der Eismaschine 1 verbunden. Die Anbindung der Steuerungseinrichtung 15 an die verschiedenen Komponenten der Vorrichtung 3 zum Desinfizieren und an die Eismaschine ist in Figur 1 durch gestrichelte Linien dargestellt.

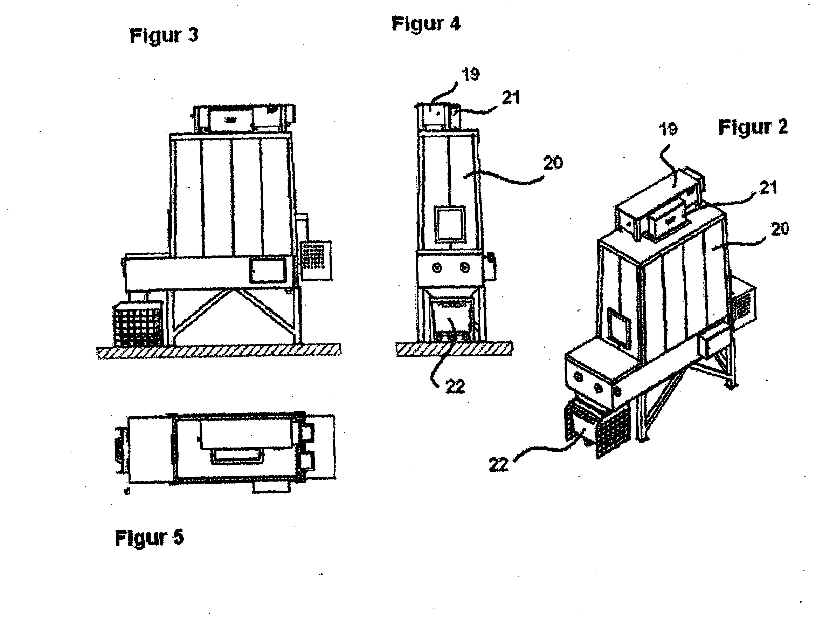

Die Figuren 2 bis 9 zeigen verschiedene Ausführungsbeispiele von Eismaschinen, Eissilos und Eisschächten, welche mit einer Vorrichtung 3 zum Desinfizieren gemäß Figur 1 ausgestattet sind. Bei dem ersten Ausführungsbeispiel gemäß den Figuren 2 bis 5 befindet sich eine Eismaschine 99 auf dem Gehäuse eines Eissilos 20. Über einen Eisschacht 21 wird das durch die Eismaschinen 19 produzierte Eis dem Eissilo zugeführt. Über das Eissilo 20 gelangt das Eis in einen Eistransportwagen 22. Die Vorrichtung zum Desinfizieren ist in das Gehäuse des Eissilos 20 integriert. Die Vorrichtung ist in den Figuren 2 bis 5 nicht erkennbar. Die Zerstäubungsdüsen sind derart in den Eisschacht 21, das Eissilo 20 und die Eismaschine 19 eingebaut, dass sich das aus der Zerstäubungsdüse expandierte Desinfektionsmittel im Inneren dieser Geräte verteilen kann.

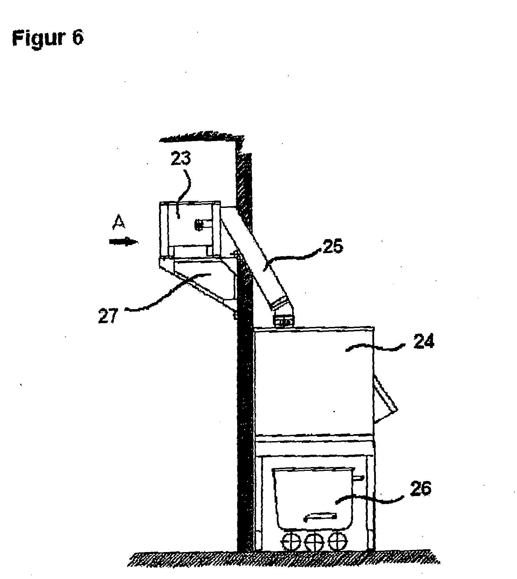

Figur 6 zeigt ein zweites Ausführungsbeispiel einer Eismaschine 23 mit einem Eissilo 24, einem Eisschacht 25 und einem Eistransportwagen 26. Im Unterschied zu dem ersten Ausführungsbeispiel gemäß der Figuren 2 bis 5 ist die Eismaschine 23 nicht auf dem Gehäuse des Eissilos 24 sondern auf einer Wandkonsole 27 angeordnet, Die Elemente einer Vorrichtung zum Desinfizieren sind in Figur 6 zwar nicht erkennbar, jedoch sind sie entsprechend dem Aufbau gemäß Figur 1 in die Bestandteile des Eissilos 24 und des Eisschachtes 25 integriert.

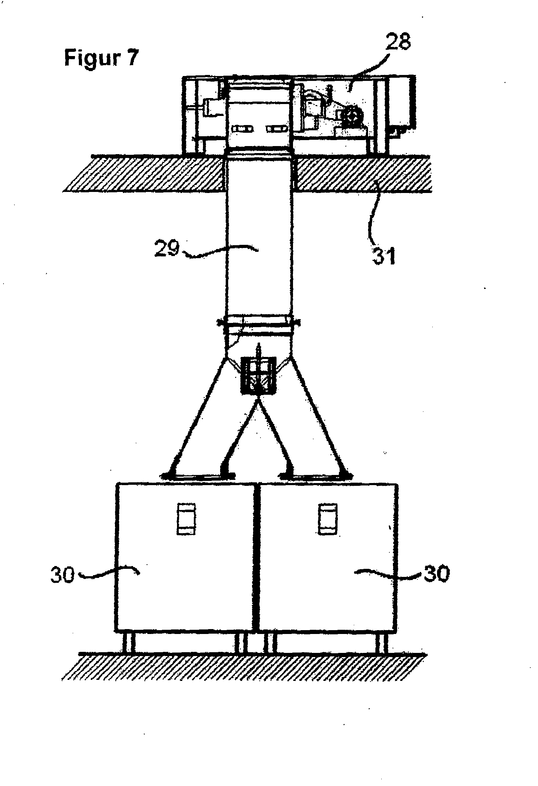

Figur 7 zeigt ein drittes Ausführungsbeispiel einer Eismaschine 28 mit einem Eisschacht 29 und zwei Eistransportwagen 30. Bei diesem Ausführungsbeispiel ist die Eismaschine 28 auf einer Zwischendecke 31 angeordnet. Bei dem Eisschacht 29 handelt es sich um einen sogenannten Hosenschacht, mit einer Klappensteuerung mit Lichtschranke. Diese sorgt dafür, dass das mit der Eismaschine 28 hergestellte Eis auf die zwei Eistransportwagen verteilt wird.

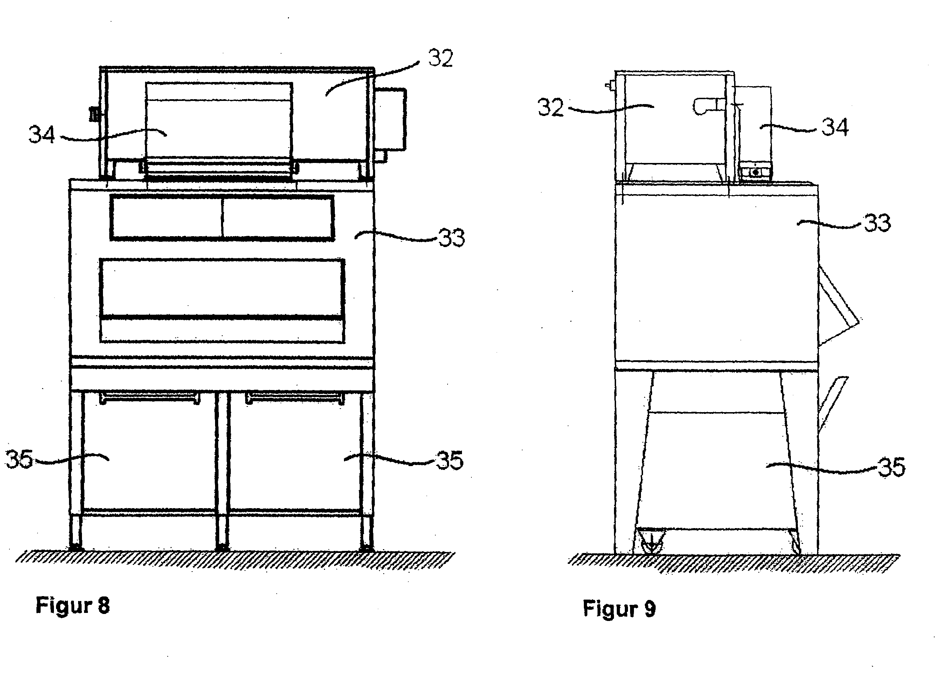

In den Figuren 8 und 9 ist ein viertes Ausführungsbeispiel mit einer Eismaschine 32, einem Eissilo 33, einem Eisschacht 34 und zwei Eistransportwagen 35 dargestellt. Das Eissilo 33 ist mit einer Klappe 36 ausgestattet, damit es zum Zwecke der Kontrolle und Instandhaltung geöffnet werden kann.

Sämtliche Merkmale der Erfindung können sowohl einzeln als auch in beliebiger Kombination miteinander erfindungswesentlich sein.

### Bezugszahlenliste

- 1: Eismaschine
- 2: Eissilo
- 3: Vorrichtung zum Desinfizieren
- 4: Eisschacht
- 5: Eistransportwagen
- 6: Zerstäubungsdüse
- 7: Druckleitung
- 8: Vorratsbehälter
- 9: Zuleitung
- 10: Druckluftkompressor
- 11: Sauglanze
- 12: Schwimmerschalter
- 13: Durchlauferhitzer
- 14: Bedieneinrichtung
- 15: Steuerungseinrichtung
- 16: Magnetventil
- 17: Druckschalter
- 18:
- 19: Eismaschine
- 20: Eissilo
- 21: Eisschacht
- 22: Eistransportwagen
- 23: Eismaschine
- 24: Eissilo
- 25: Eisschacht
- 26: Eistransportwagen
- 27: Wandkonsole
- 28: Eismaschine
- 29: Eisschacht
- 30: Eistransportwagen
- 31: Zwischendecke
- 32: Eismaschine
- 33: Eissilo
- 34: Eisschacht
- 35: Eistransportwagen

## Patentansprüche

1. Vorrichtung zum Desinfizieren von Eismaschinen, Eissilos und/ oder Kanälen zum Transportieren von Eis
mit einem Vorratsbehälter (8) zur Aufnahme eines Desinfektionsmittels,
mit einer Druckleitung (7) zum Zuleiten eines Trägerfluids unter Druck,
mit mindestens einer Zersiäubungseinrichtung (6) zum Zerstäuben des Desinfektionsmittels und zur Erzeugung eines Gemischs aus Trägerfluid und Desinfektionsmittel und
mit einem Einlass zum Einleiten des Trägerfluid-Desinfektionsmittel-Gemischs in die Eismaschine (1,19,23,28, 32), das Eissilo (2, 20, 24, 33) oder die Eiskanäle (4, 21, 29, 34).

2. Vorrichtung nach Anspruch 1, **dadurch gekennzeichnet, dass** die Zerstäubungseinrichtung eine Zerstäubungsdüse ist.

3. Vorrichtung nach Anspruch 1, **dadurch gekennzeichnet, dass** die Zerstäubungseinrichtung ein Kontaktzerstäuber, ein Rieselzerstäuber, ein Scheibenzerstäuber, ein Dampfzerstäuber, ein Ultraschallzerstäuber oder ein Klingenburg-Zerstäuber ist.

4. Vorrichtung nach einem der vorhergehenden Ansprüche, **dadurch gekennzeichnet, dass** die mit der Zerstäubungseinrichtung zerstäubten Partikel des Trägerfluids einem Durchmesser im Mittel von weniger als 200µm, besonders bevorzugt von weniger als 100µm aufweisen.

5. Vorrichtung nach einem der vorhergehenden Ansprüche, **dadurch gekennzeichnet, dass** der Vorratsbehälter (8) mit Wasserstoffperoxid als Desinfektionsmittel ausgestattet ist.

6. Vorrichtung nach einem der vorhergehenden Ansprüche, **dadurch gekennzeichnet, dass** der Vorratsbehälter (8) mit der Zerstäubungseinrichtung (6) über eine Desinfektionsmittel-Zuleitung (9) verbunden ist.

7. Vorrichtung nach Anspruch 1 oder 6, **dadurch gekennzeichnet, dass** an der Zerstäubungseinrichtung (6) und/ oder an der Desinfektionsmittel-Zuleitung (9) eine Heizeinrichtung (13) angeordnet ist.

8. vorrichtung nach einem der vorhergehenden Ansprüche, **dadurch gekennzeichnet, dass** an der Druckleitung (7) eine Heizeinrichtung angeordnet ist.

9. Vorrichtung nach einem der vorhergehenden Ansprüche, **dadurch gekennzeichnet, dass** an der Druckleitung (7) zum Zuleiten des Trägerfluids ein Kompressor (10) angeordnet ist.

10. Vorrichtung nach einem der vorhergehenden Ansprüche, **dadurch gekennzeichnet, dass** das Trägerfluid Luft ist.

11. Vorrichtung nach einem der vorhergehenden Ansprüche, **dadurch gekennzeichnet, dass** sie mit einer Dosiereinrichtung zum Dosieren des Desinfektionsmittels in das Trägerfluid ausgestattet ist.

12. Vorrichtung nach einem der vorhergehenden Ansprüche, **dadurch gekennzeichnet, dass** sie mit einer Mischeinrichtung zum Mischen des Desinfektionsmittels in dem Trägerfluid ausgestattet ist.

13. Vorrichtung nach Anspruch 2, **dadurch gekennzeichnet, dass** die Zerstäubungsdüse (6) einen Strömungskanal aufweist, in welchen die Druckleitung (7) mündet, dass an dem der Druckleitung (7) abgewandten Ende des Strömungskanals die Düsenöffnung angeordnet ist, und dass der Strömungskanal über eine Zuleitung oder eine Dosiereinrichtung mit dem Vorratsbehälter (8) des Desinfektionsmittels verbunden ist.

14. Vorrichtung nach Anspruch 13, **dadurch gekennzeichnet, dass** der Strömungskanal mit einem Hindernis zur Erzeugung von Verwirbelungen ausgestattet ist.

15. Verfahren zum Desinfizieren von Eismaschinen, Eissilos und/ oder Eiskanälen zum Transportieren von Eis, insbesondere unter Verwendung einer Vorrichtung nach einem der Ansprüche 1 bis 14, **gekennzeichnet durch** folgende Verfahrensschritte:
dass ein Trägerfluid unter einem Druck zugeleitet wird, der oberhalb des Atmosphärendrucks liegt,
dass ein Desinfektionsmittel mit dem Trägerfluid gemischt wird
dass das Trägerfluid -Desinfektionsmntel-Gemisch mittels einer Zerstäubungseinrichtung (6) fein zerstäubt wird, wobei der Durchmesser der Pratikel kleiner als 100µm ist,
dass das zerstäubte Trägerfluid-Desinfektionsmittel-Gemisch in die Eismaschine, das Eissilo und/ oder die Eiskanäle zum Transportieren des Eises expandiert wird.

16. Verfahren nach Anspruch 15, **dadurch gekennzeichnet, dass** als Desinfektionsmittel Wasserstoffperoxid eingesetzt wird.

17. Verfahren nach Anspruch 15 oder 16, **dadurch gekennzeichnet, dass** als Trägerfluid Luft eingesetzt wird.

18. Verfahren nach einem der Ansprüche 15 bis 17, **dadurch gekennzeichnet, dass** das Desinfektionsmittel, das Trägerfluid oder das Trägerfluid-Desinfektionsmittel-Gemisch vor dem Expandieren auf eine Temperatur oberhalb der Umgebungstemperatur erwärmt wird.

19. Eismaschine zur Herstellung von Eis in kleinen Stücken, **dadurch gekennzeichnet, dass** sie mit einer Vorrichtung nach einem der Ansprüche 1 bis 14 ausgestattet ist.

20. Eissilo zum Sammeln und Lagern von Eis in kleinen Stücken, **dadurch gekennzeichnet, dass** es mit einer Vorrichtung nach einem der Ansprüche 1 bis 14 ausgestattet ist.
